# EUROPEAN PATENT SPECIFICATION

(11) **EP 3 585 269 B1**
(45) Date of publication and mention of the grant of the patent: **28.07.2021**
(21) Application number: 18715476.0
(22) Date of filing: 22.02.2018
(51) Int. Cl.: G16H 40/63, A61B 8/00, G16H 40/67

(54) **REMOTELY CONTROLLED ULTRASONIC IMAGING SYSTEM**
FERNGESTEUERTES ULTRASCHALLBILDGEBUNGSSYSTEM
SYSTÈME D'IMAGERIE PAR ULTRASONS COMMANDÉ À DISTANCE

(30) Priority: 22.02.2017 US 201762461960 P
(43) Date of publication of application: 01.01.2020
(73) Proprietor: Koninklijke Philips N.V., 5656 AG Eindhoven (NL)
(72) Inventor: LEYVI, Evgeniy, 5656 AE Eindhoven (NL); RAJU, Balasundar Iyyavu, 5656 AE Eindhoven (NL); WANG, Shougang, 5656 AE Eindhoven (NL); ZHOU, Shiwei, 5656 AE Eindhoven (NL); SOOMRO, Amjad, 5656 AE Eindhoven (NL); HEGDE, Sanjay Ramachandra, 5656 AE Eindhoven (NL)
(74) Representative: Philips Intellectual Property & Standards
(86) International application number: PCT/EP2018/054375
(87) International publication number: WO 2018/153979

(56) References cited:
- WO-A1-2017/013511
- US-A1- 2003 083 563
- US-A1- 2015 015 379

## Description

This invention relates to medical diagnostic ultrasound systems and, in particular, to the use of ultrasound systems for remote diagnosis with remote system control.

Currently available medical ultrasound systems enable clinicians to conduct ultrasound scans on a patient, capture images, make measurements and use built-in algorithms and report generation software to make diagnoses and report the results of a diagnosis. The clinicians who perform these procedures are experienced radiologists, cardiologists, obstetricians, or trained sonographers. Sufficient training is expected before a clinician can conduct the procedure and interpret the scanned images. However, such medical experts may not be readily available in a remote area. One approach to providing necessary diagnostic care is teleradiology, whereby an inexperienced person at the location of the patient is guided in the conduct of a scan by a remotely located medical expert. Teleradiology can not only be used to provide diagnostic imaging services to remote areas where skilled diagnostic personnel are not otherwise available. It can also be used to provide convenience and reduce costs for patients who need frequent scanning such as those at high risk for certain conditions or patients who have recently undergone a procedure which requires periodic follow-up monitoring. In the usual diagnostic scenario, ultrasound scanning is done by an experienced sonographer at a facility with a variety of ultrasound systems for different diagnostic procedures. The patient must travel to the diagnostic facility which often may be inconvenient or difficult for the patient. Moreover, the patient or his or her insurance company can incur considerable expense for scanning performed at such highly equipped facilities staffed by expert sonographic personnel. These costs and inconvenience can mount when a patient requires frequent scanning to monitor an ongoing medical condition, for instance.

An approach which reduces such costs and inconvenience is to have the scanning performed by someone who is not an experienced sonographer but under the direction of a remotely located clinician who is in communication with the person performing the scan. That person may be a visiting nurse, a caregiver, or even the patient himself. Using teleradiology, the images produced by the scan at the patient site are viewed by the remote expert, who assures that the proper diagnostic images are acquired and subsequently reviewed. When teleradiology is employed in this way, the patient does not have to travel to a hospital or diagnostic imaging facility, and the costs incurred by the reviewing facility are minimized.

Providing ultrasound scanning in this way does require that the ultrasound scanner be present at the patient site such as the home of the patient, however. The costs attendant to providing an ultrasound scanner for patient use can be minimized by using a compact, highly portable scanner, such as one of the Lumify™ ultrasound probes available from Philips Healthcare of Andover, MA. The Lumify probes are designed for specific scanning procedures such as curved array probes for radiological procedures and phased array probes for cardiovascular procedures. These probes contain all of the circuitry necessary to produce an ultrasound image in the probe case itself. A Lumify probe thus needs only a display device to complete a fully functional ultrasonic diagnostic imaging system. The display device can be anyone of commonly available desktop, laptop or tablet computers or even a standard smartphone. The software necessary to interface a Lumify probe to one of these standard devices can be downloaded from the user's application provider. Once an app is downloaded and installed, the Lumify probe and the user's display device form a fully functional ultrasound system.

US 2003/083563 A1 discloses a system for streaming unprocessed medical image data from a medical imaging system to a remote terminal, in which the remote terminal receives the unprocessed medical image data, processes it to render a medical image and displays the medical image to an operator at the remote terminal.

US 2015/015379 A1 relates to a method of controlling an image diagnosis apparatus by using a mobile terminal, in which control commands from the outside can be input in the mobile terminal through a virtual keypad and transmitted to the image diagnosis apparatus via a Wi-Fi direct or ultra wideband network.

WO 2017/013511 A1 describes a highly portable ultrasound system formed by a wireless ultrasound probe, a processor dongle containing a radio and a digital processor running an operating system and an ultrasound control program, and a display monitor. The sonographer only needs to carry the small wireless probe and the thumbdrive-like dongle in order to turn any available display device, together with the two components carried by the sonographer, into a completely functional ultrasound system.

Conventionally, fully functional ultrasound systems are always under the control of medical professionals who take great pains to ensure that they are always used properly. One guiding principle in the use of ultrasound by ultrasound professionals is the ALARA principle. This acronym stands for "as low as reasonably achievable," which means that the ultrasound professional uses ultrasound in a way which achieves the diagnostic objective while exposing the patient to the minimal amount of ultrasonic energy necessary to provide the required images. While ultrasound uses non-ionizing radiation and is generally safe under virtually all conditions, including unborn infants, the ALARA principle is nonetheless followed by all responsible ultrasound diagnosticians. But when an ultrasound system is left in the hands of someone inexperienced in ultrasound or the patient himself, this guiding principle may not always be followed. The patient or the person doing the scanning may use the system to perform unauthorized scanning, not only of the patient but even of other persons. Using conventional preventive measures such as passwording the ultrasound system may not be fully effective. Once the patient or caregiver has received the password for the initial scanning procedure, there is no way to prevent its use for subsequent unauthorized scanning. It is therefore desirable to provide an ultrasound system which can be used by laypersons or caregivers to scan a person at home or another location outside a diagnostic facility, but which still prevents any use of the ultrasound system for unauthorized scanning.

Accordingly, it is an object of the present invention to make ultrasound imaging available at the site of a patient with the help of an expert at a remote location. It is a further object to do so in a way that facilitates local scanning with remote expert assistance without also facilitating improper use of the scanning device such as unauthorized scanning with the device. A solution which meets these objectives will enable diagnostic imaging in locations where diagnostic imaging experts are not readily available for cost or even reasons of convenience.

The above objects are solved by the ultrasound imaging system of Claim 1. Advantageous embodiments are defined in the dependent claims.

In accordance with the principles of the present invention a system and method are described which enable ultrasonic imaging by inexperienced personnel under the guidance of a remotely located diagnostic imaging expert. Improper use of the scanning device is prevented by having the remote expert control the operation of the ultrasound system, including such operation as enabling or disabling ultrasound energy transmission by an ultrasound probe and enabling or disabling imaging on the ultrasound system. The remote expert can thereby direct and control a scanning procedure, remotely enabling the ultrasound system when an authorized scan is to be performed, viewing images from the ultrasound exam on his or her image display, changing scan settings if needed, and limiting the local display and/or disabling the ultrasound system when the authorized procedure is completed.

In the drawings:
FIGURE 1 illustrates an ultrasound scanning device and a remote expert diagnostic workstation configured in accordance with the principles of the present invention.
FIGURE 2 illustrates in block diagram form an ultrasound system configured for use by an untrained user an under control of a remote diagnostic clinician in accordance with the principles of the present invention.
FIGURE 2a illustrates in detail the command decoder circuitry of FIGURE 2.
FIGURE 3 is a flowchart illustrating the sequence of a scanning procedure using an ultrasonic scanning device at the site of a patient and a remotely located expert in accordance with the present invention.

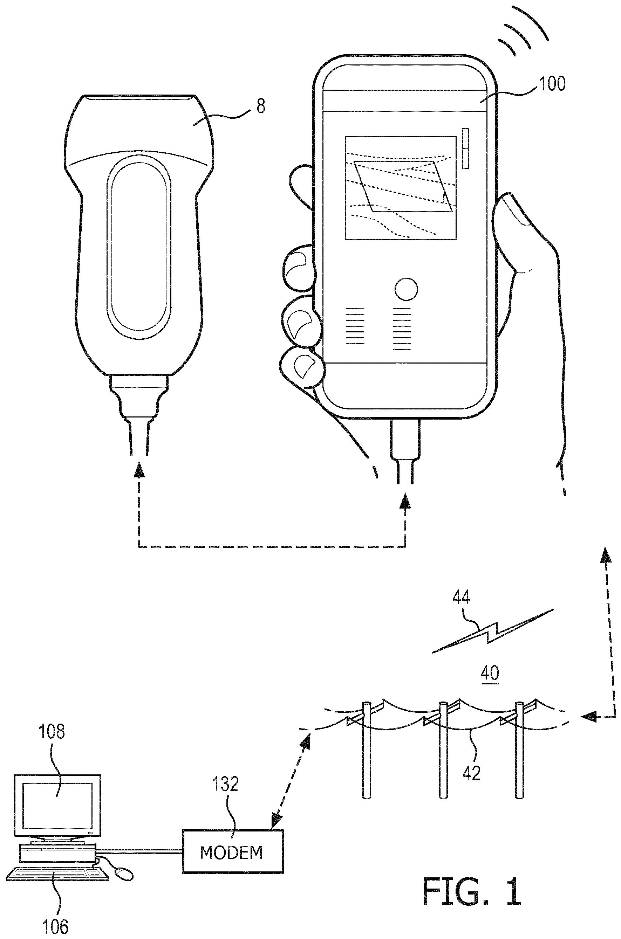

FIGURE 1 illustrates at the top of the drawing a highly portable ultrasonic scanning device comprising an L12-5 Lumify™ ultrasound probe 8 coupled to a handheld display device in the form of a smartphone 100. The Lumify ultrasound system, available from Philips Healthcare of Andover, MA, USA, consists of a Lumify probe such as the one shown in the drawing, and an image display device, which may comprise a PDA, laptop computer, desktop computer, tablet computer, or smartphone 100 as shown in this illustration. In this system all of the ultrasound-specific components, such as the transducer array, beamformer, ultrasound signal processor, and B mode and Doppler processors, are constructed in piezoelectronic and integrated circuit form and located in the probe 10. The display device 100 contains the image display and the user interface (control) components. The display device also contains a software application specific to the Lumify probe, which causes the ultrasound image and the user controls to be displayed on the device screen as shown in the drawing. In this smartphone implementation the user holds the ultrasound probe 8 against the skin of the patient with one hand while holding the display and user control device 100 with the other hand. The acquired ultrasound images are displayed on the display device, and the same images are sent wirelessly to the display device of a remote expert. In the illustrated implementation the expert's ultrasound image display device is the screen of a workstation 108 which is controlled by a keyboard 106. The expert sends commands to the scanning device from the workstation and receives images from the scanning device by means of a modem or wireless radio 132 which are carried over a data network 40, e.g., the Internet, which may take the form of a wire network 42 or a wireless network as indicated by 44.

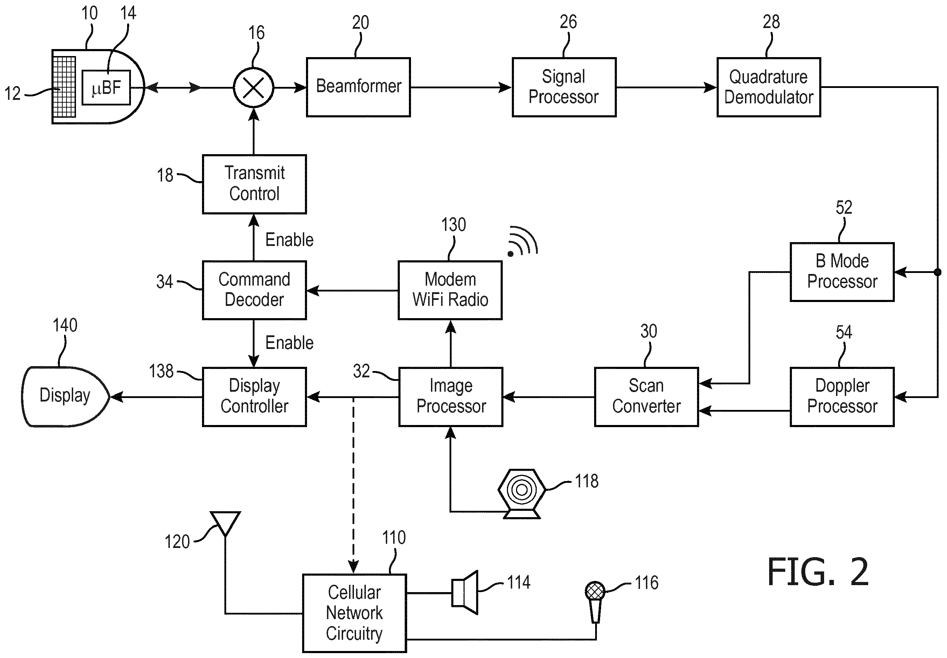

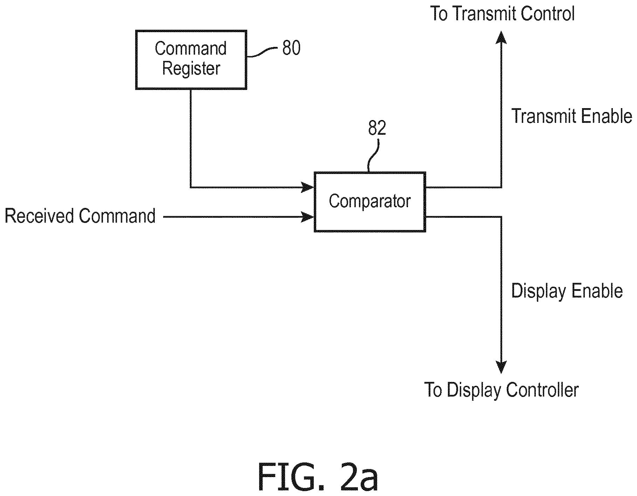

FIGURE 2 illustrates the components of an exemplary ultrasonic scanning device in block diagram form. A transducer array 12 is provided in an ultrasound probe 10 for transmitting ultrasonic waves and receiving echo information. The transducer array 12 may be a one- or two-dimensional array of transducer elements capable of scanning in two or three dimensions, for instance, in both elevation (in 3D) and azimuth. The transducer array 12 is coupled to a microbeamformer 14 in the probe which controls transmission and reception of signals by the array elements. Microbeamformers are capable of at least partial beamforming of the signals received by groups or "patches" of transducer elements as described in US Pats. 5,997,479 (Savord et al.), 6,013,032 (Savord), and 6,623,432 (Powers et al.) The microbeamformer is coupled to a transmit/receive (T/R) switch 16 which switches between transmission and reception and protects the main beamformer 20 from high energy transmit signals. In the event that the microbeamformer 14 performs all of the beamformation, as it does in the Lumify probe, then a main beamformer is unnecessary. The transmission of ultrasonic beams from the transducer array 12 under control of the microbeamformer 14 is directed by a transmit controller 18 coupled to the T/R switch and the microbeamformer 14, which receives input from the user's operation of a user interface or control panel (not shown) and, in this implementation of the present invention, receives an "enable" signal from a command decoder 34. Among the transmit characteristics controlled by the transmit controller are the timing, amplitude, phase, and polarity of transmit waveforms. Beams formed in the direction of pulse transmission may be steered straight ahead from (orthogonal to) the transducer array, or at different angles for a wider field of view. They may also be focused at different depths in the body of a subject.

The echoes received by a group of transducer elements are beamformed by appropriately delaying them and then combining them. The partially beamformed signals produced by the microbeamformer 14 from each patch are coupled to a main beamformer 20 where partially beamformed signals from individual patches of transducer elements are combined into a fully beamformed coherent echo signal. For example, the main beamformer 20 may have 128 channels, each of which receives a partially beamformed signal from a patch of 12 transducer elements. In this way the signals received by over 1500 transducer elements of a two-dimensional array can contribute efficiently to a single beamformed signal.

The coherent echo signals undergo signal processing by a signal processor 26, which includes filtering by a digital filter and noise reduction as by spatial or frequency compounding as shown in US Pats. 4,561,019 (Lizzi et al.) and 6,390,981 (Jago), for instance. The signal processor can also shift the frequency band to a lower or baseband frequency range. The digital filter of the signal processor 26 can be a filter of the type disclosed in U.S. Patent No. 5,833,613 (Averkiou et al.), for example. The processed echo signals then are demodulated into quadrature (I and Q) components by a quadrature demodulator 28, which provides signal phase information.

The beamformed and processed coherent echo signals are coupled to a B mode processor 52 which produces a B mode tissue image. The B mode processor performs amplitude (envelope) detection of quadrature demodulated I and Q signal components by calculating the echo signal amplitude in the form of (I²+Q²)^{½}. The quadrature echo signal components are also coupled to a Doppler processor 54, which stores ensembles of echo signals from discrete points in an image field which are then used to estimate the Doppler shift at points in the image with a fast Fourier transform (FFT) processor. For a color Doppler image as shown on the display device 100 of FIGURE 1, the estimated Doppler flow values at each point in a blood vessel are wall filtered and converted to color values using a look-up table. The B mode image signals and the Doppler flow values are coupled to a scan converter 30 which converts the B mode and Doppler samples from their acquired R-θ coordinates to Cartesian (x,y) coordinates for display in a desired display format, e.g., a sector display format or a rectilinear display format as shown in FIGURE 1. Either the B mode image or the Doppler image may be displayed alone, or the two shown together in anatomical registration in which the color Doppler overlay shows the blood flow in the structure of tissue and vessels in the image.

The ultrasound images produced by the scan converter 30 are coupled to an image processor 32. The image processor may further smooth and filter the image for display, and add graphical information such as patient name, date, and scanning parameters. The image processor may also convert 3D data sets or sets of image planes into three dimensional images by volume rendering as described in US Pat. 6,530,885 (Entrekin et al.), or extract individual image planes from a 3D data set by multiplanar reformatting as described in US Pat. 6,443,896 (Detmer). For local display on the display device the processed images are coupled to the display device's display controller 138, which displays the images on a display 140.

In an implementation of the present invention the ultrasound images produced by the image processor 32 are coupled to a MODEM or WiFi radio 130 for transmission to the display device of a remote expert. The MODEM or WiFi 130 can be the conventional WiFi transceiver found in smartphones, laptop, tablet and desktop computers. In addition to transmitting ultrasound images to the remote expert, the MODEM/WiFi radio 130 also receives commands transmitted by the remote expert to control the ultrasound functionality of the system of FIGURE 2. The received commands, generally in the form of digital words, are coupled to a command decoder 34 which decodes the commands into signals which control the ultrasound system. In the implementation of FIGURE 2, a command which enables ultrasound transmission from the transducer array 12 decodes to produce an "enable" signal that is applied to the transmit controller 18. The transmit controller, microbeamformer 14, and transducer array 12 are then enabled to transmit ultrasonic energy. When the enable signal is changed to disable by another command, the transmit controller 18 and transducer array 12 cease ultrasound transmission. Also shown in the drawing is an enable signal produced by decoding of a command to enable local display of ultrasound images. When this enable signal is produced and applied to the display controller 138, the display controller will display the ultrasound images produced by the system on the local display 140. When another command disables the enable signal, the display controller is inhibited from displaying the ultrasound images on the local display 140. In a typical implementation the command decoder 34 receives a variety of commands from the remote expert to control an ultrasound scan, in addition to the two shown in FIGURE 2. Other commands which may be sent to the ultrasound system include those to select or change the imaging mode, e.g., changing from B mode to colorflow mode and back again, or adjusting the focal depth of the image. The command set generally would include those used in the conduct of a usual ultrasound exam.

When the ultrasound system display device is a smartphone 100 as shown in FIGURE 1, it contains cellular network circuitry 110 for wireless transmission in addition to WiFi communication over the Internet. In the implementation shown in FIGURE 2, audio communication is done over a cellular network. This includes voice input from the smartphone's microphone 116 which is coupled to the cellular networks circuitry and transmitted by means of antenna 120, and voice communication from the remote expert which is received over the cellular network and reproduced on the smartphone's loudspeaker 114. The smartphone's camera 118, which in the case of a desktop computer implementation would comprise a Webcam, is coupled to the image processor 32 so that live images can be sent to the remote expert via the MODEM/WiFi radio 130. Thus, in the implementation shown in FIGURE 2, video communication is via WiFi, and audio communication is via cellular network. However, this partitioning of communication in a particular implementation is a matter of design choice; all communications can be via WiFi, via cellular network, or other partitioning thereof. The example of FIGURE 2 shows a dotted line from the output of the image processor 32 to the cellular network circuitry 110, which would be used to conduct both audio and video communication over the cellular network. The cellular network circuitry can also be configured to receive the control commands sent from the clinician's remote location for the command decoder 34.

The structure of one implementation of the command decoder 34 is shown in FIGURE 2a. A received digital command word is coupled to a digital comparator 82. The comparator sequentially compares the received command with command words stored in a command register 80, implemented as a digital memory, each of which can be implemented by the ultrasound system to produce a known control effect. When a match is found of a received command with one stored in the command register, the control function of the identified command is implemented by the ultrasound system. For example, when the received command word matches with the Transmit Enable command word from the command register, the comparator initiates the application of a transmit enable signal to the transmit controller 18 as shown in FIGURE 2, which enables the transmission of ultrasound energy by the transducer array. Using this command, a remote expert can enable the ultrasound probe in the hands of a patient or layperson for ultrasonic imaging. When the imaging procedure is completed, the remote expert can send a similar Transmit Disable command which, when matched with the known Transmit Disable command from the command register, disables the ultrasound probe from further ultrasonic energy transmission. By enabling the ultrasound probe to transmit ultrasound only for a legitimate imaging exam, the remote expert assures adherence to the ALARA principle in the use of the system in the hands of a layperson.

Similarly, when the received command matches with the Display Enable command from the command register, the comparator initiates the application of a display enable signal to the display controller 138 as shown in FIGURE 2. Using this command the remote expert can control when the ultrasound images produced during the scan will be shown to individuals at the site of the scanning, and when display of the images will be inhibited. Many countries have laws in place to restrict the use of ultrasound for use by an expert for medical diagnosis purpose only. In some countries gender identification is an issue and it is illegal to determine gender of a fetus before birth using ultrasound. Only licensed persons can perform ultrasound imaging. If an ultrasound system is provided to a patient for legitimate use such as monitoring the condition of the patient after surgery, it is conceivable that the device could be misused for gender identification purposes. One approach to preventing such misuse is to block the display of images to unqualified or unauthorized users at the scanning site, and allowing only the remote expert to view the ultrasound images. The implementation of FIGURE 2 provides this capability.

In the Lumify system of FIGURE 1, the display controller 138 and the display 140, the MODEM/WiFi radio 130, the camera 118, microphone 116, loudspeaker 114, and cellular network circuitry 110 and antenna 120 are located in the smartphone display unit 100, while the other components of FIGURE 2 are located in the probe housing 8. The user controls on the touchscreen display of the smartphone display unit, not shown in FIGURE 2, can also send control signals to the probe unit 8, which appropriately control the other components of the scanning system in accordance with the user's manipulation of the controls. Other implementations, for instance, ones using a tablet or laptop computer as the display device, may partition the components differently so that more components are located in the computer.

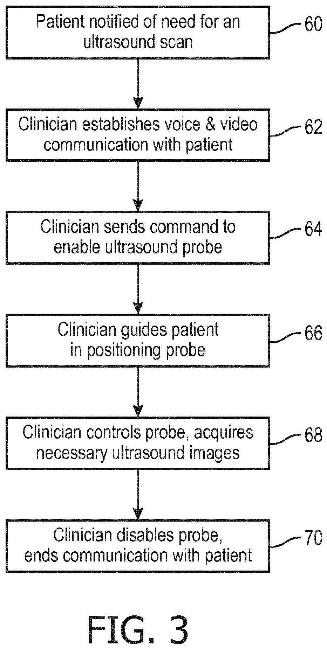

A method for use of the ultrasound scanning device of FIGURE 1 by an untrained user to perform scanning with the probe, with control of the procedure by a remote expert clinician at the image workstation 106, 108, is outlined by the flowchart of FIGURE 3. In step 60 a patient is notified of the need for an ultrasound scan. This notification may be by phone or email or text message about a need for an ultrasound scan either because a patient developed symptoms described by the patient to the medical professional and which, in the judgment of the medical professional, require an ultrasound examination or because time has come for a scheduled ultrasound monitoring examination, such as monitoring a post-surgery patient for possible internal bleeding or fluid build-up. When a person at the patient site, such as the patient himself or herself, is ready to conduct the necessary scan, the clinician establishes voice and video communication in step 62 with the patient and/or the person who will scan the patient. The video can be provided for instance by the camera of the smartphone or by a Webcam, and the audio can be over a cellular network of using a voice-over-IP protocol communicating over the Internet. In step 64 the remote clinician sends a command from the workstation 106, 108 to enable the transmission of ultrasound by the ultrasound probe 8. In step 66 the remote clinician then guides the person holding the ultrasound probe, the patient in this example, in properly positioning the probe against the body of the patient so that the desired anatomy is in the image field. This is done in this example by observing the probe placement with the live images transmitted by the smartphone camera or a Webcam. Once the remote clinician is seeing ultrasound images of the proper anatomy on the workstation, images which may or may not be visible to the person(s) at the scanning site depending upon transmission of a Display Enable command, the remote clinician will begin carefully observing the ultrasound images and generally recording them. In step 68 the remote clinician sends additional commands to the ultrasound system to optimize the exam, such as mode change commands and focal depth change commands, and may issue additional verbal instructions to guide probe manipulation. The exam is complete when the clinician has acquired and stored the necessary images for a diagnosis. With the exam completed, the remote expert sends a command to disable further ultrasound transmission by the probe in step 70 and ends the voice and video communication with the patient and others at the scanning site.

Variations of the system and method described above will readily occur to those skilled in the art. Instead of enabling or disabling the transmit controller to control the production of ultrasonic energy by the probe, the enabling command may enable an ultrasound image acquisition program executed by the ultrasound system for image acquisition. Alternatively, the image acquisition program may be downloaded to the ultrasound system under control of the remote clinician, then removed from the ultrasound system after the ultrasound exam is completed. Some or all of the image acquisition program may be resident on the cloud and executed there in whole or in part and never be fully loaded onto the ultrasound system in the hands of a layperson scanner. Instead of enabling and disabling the transmit controller, the enable command may control the application of high voltage to the transducer drivers in the microbeamformer by closing (and later opening) a switch in the high voltage supply line. Instead of using command words, an enabling code may be verbally given to the person conducting the scan to input into the ultrasound system, provided that the code is only effective for a single scan and thus cannot be misused at a later time.

It should be noted that an ultrasound system suitable for use in an implementation of the present invention, and in particular the component structure of the workstation and ultrasound systems of FIGURES 1 and 2, may be implemented in hardware, software or a combination thereof. The various embodiments and/or components of an ultrasound system, for example, the modules, or components and controllers therein, also may be implemented as part of one or more computers or microprocessors. The computer or processor may include a computing device, an input device, a display unit and an interface, for example, for accessing the Internet as shown in FIGURE 1. The computer or processor may include a microprocessor. The microprocessor may be connected to a communication bus, for example, to access a PACS system or the data network shown in FIGURE 1. The computer or processor may also include a memory. The memory devices such as the command register 80 may include Random Access Memory (RAM) and Read Only Memory (ROM). The computer or processor further may include a storage device, which may be a hard disk drive or a removable storage drive such as a floppy disk drive, optical disk drive, solid-state thumb drive, and the like. The storage device may also be other similar means for loading computer programs or other instructions into the computer or processor.

As used herein, the term "computer" or "module" or "processor" or "workstation" may include any processor-based or microprocessor-based system including systems using microcontrollers, reduced instruction set computers (RISC), ASICs, logic circuits, and any other circuit or processor capable of executing the functions described herein. The above examples are exemplary only, and are thus not intended to limit in any way the definition and/or meaning of these terms.

The computer or processor executes a set of instructions that are stored in one or more storage elements, in order to process input data. The storage elements may also store data or other information as desired or needed. The storage element may be in the form of an information source or a physical memory element within a processing machine.

The set of instructions of an ultrasound system including those controlling the acquisition, processing, and transmission of ultrasound images as described above may include various commands that instruct a computer or processor as a processing machine to perform specific operations such as the methods and processes of the various embodiments of the invention. The set of instructions may be in the form of a software program. The software may be in various forms such as system software or application software and which may be embodied as a tangible and non-transitory computer readable medium. Further, the software may be in the form of a collection of separate programs or modules, a program module within a larger program or a portion of a program module. The software also may include modular programming in the form of object-oriented programming. The processing of input data by the processing machine may be in response to operator commands, or in response to results of previous processing, or in response to a request made by another processing machine. In the Lumify system smartphone shown in FIGURE 1, for instance, software instructions are conventionally employed to create and control the display and user control functions described above and are downloaded to the smartphone as an app (application software.)

## Claims

1. An ultrasound imaging system for remote control of a scanning procedure comprising:
an ultrasound scanning device adapted to be coupleable with a local display and user control device (100) for control by a local user, and comprising an ultrasound probe (8, 10) adapted to controllably transmit ultrasound energy, an ultrasound image processor (32), and a communication unit adapted to send image data to and receive control signals from a remote location connected to the ultrasound scanning device via a network connection (40);
an ultrasound image display (108) that is located at the remote location and adapted to display the image data received from the ultrasound scanning device, wherein the ultrasound scanning device is adapted to be responsive to control signals initiated from the remote location, including an enable and disable command signal configured to enable and disable the transmission of ultrasound energy by the ultrasound probe (8, 10) while under the operation of the local user.

2. The ultrasound imaging system of Claim 1, wherein the ultrasound scanning device is further adapted to be responsive to commands sent from the remote location to control the ultrasound scanning device.

3. The ultrasound imaging system of Claim 2, wherein the enable and disable command signals are digital commands sent from the remote location to enable and disable the transmission of ultrasound energy by the ultrasound scanning device.

4. The ultrasound imaging system of Claim 3, wherein the ultrasound scanning device further comprises a command decoder (34) that is responsive to digital commands sent from the remote location and adapted to determine the identity of received commands.

5. The ultrasound imaging system of Claim 2, wherein the ultrasound scanning device is further adapted to be responsive to verbal commands sent from the remote location to enable the ultrasound scanning device to transmit ultrasound energy for the instantaneous scanning procedure.

6. The ultrasound imaging system of Claim 1, wherein the ultrasound scanning device further comprises a transmit controller (18) that is coupled to the ultrasound probe (8, 10) and adapted to be responsive to the reception of the enable and disable signals initiated from the remote location to enable and disable the transmission of ultrasound energy by the ultrasound probe.

7. The ultrasound imaging system of Claim 6, wherein the ultrasound probe (8, 10) further comprises a transducer array (12) adapted to transmit ultrasound energy under control of the transmit controller (18).

8. The ultrasound imaging system of Claim 1, wherein the ultrasound scanning device further comprises a MODEM/WiFi radio (130) adapted to send images to the remote location and receive control commands from the remote location.

9. The ultrasound imaging system of Claim 1, wherein the ultrasound scanning device further comprises cellular network circuitry (110) adapted to send images to the remote location and receive control commands from the remote location.

10. The ultrasound imaging system of Claim 1, wherein the ultrasound scanning device is further adapted for video communication with the remote location.

11. The ultrasound imaging system of Claim 10 wherein the ultrasound scanning device further comprises a smartphone camera (118) or a Webcam.

12. The ultrasound imaging system of Claim 1, wherein the ultrasound scanning device is further adapted for audio communication with the remote location.

13. The ultrasound imaging system of Claim 12, wherein the ultrasound scanning device further comprises a loudspeaker (114) and a microphone (116), wherein preferably the ultrasound scanning device further comprises an ultrasound image acquisition program which is adapted to be enabled or disabled from the remote location.

14. The ultrasound imaging system of Claim 1, wherein the ultrasound probe (8, 10) further comprises an array transducer (12) and a high voltage supply selectively coupled to the array transducer,
wherein the high voltage supply is adapted to be selectively coupled to the array transducer under control from the remote location.

15. Computer implemented method for remote control of a scanning procedure by means of an ultrasound imaging system, comprising:
coupling of an ultrasound scanning device with a local display and user control device (100) for control by a local user;
controllably transmitting ultrasound energy by means of an ultrasound probe (8, 10) adapted to controllably transmit ultrasound energy;
sending, by means of an ultrasound image processor (32) and a communication unit, image data to and receiving control signals from a remote location connected to the ultrasound scanning device via a network connection (40);
displaying the image data received from the ultrasound scanning device by means of an ultrasound image display (108) that is located at the remote location;
responding, by the ultrasound scanning device, to control signals initiated from the remote location including an enable and disable command signal configured to enable and disable the transmission of ultrasound energy by the ultrasound probe (8, 10) while under the operation of the local user.

## Patentansprüche

1. Ultraschallbildgebungssystem zur Fernsteuerung eines Abtastverfahrens, umfassend:
eine Ultraschallabtastvorrichtung, die angepasst ist, um mit einer lokalen Anzeige- und Benutzersteuervorrichtung (100) zur Steuerung durch einen lokalen Benutzer koppelbar zu sein, und umfassend eine Ultraschallsonde (8, 10), die zur steuerbaren Übertragung von Ultraschallenergie geeignet ist, einen Ultraschallbildprozessor (32) und eine Kommunikationseinheit, die angepasst ist, um Bilddaten an einen entfernten Ort zu senden und Steuersignale von einem entfernten Ort zu empfangen, der über eine Netzwerkverbindung (40) mit der Ultraschallabtastvorrichtung verbunden ist;
eine Ultraschallbildanzeige (108), die sich an dem entfernten Ort befindet und angepasst ist, um die von der Ultraschallabtastvorrichtung empfangenen Bilddaten anzuzeigen, wobei die Ultraschallabtastvorrichtung angepasst ist, um auf Steuersignale zu reagieren, die von dem entfernten Ort initiiert wurden, einschließlich einer Aktivierung und Deaktivierung des Befehlssignals, das konfiguriert ist, um die Übertragung von Ultraschallenergie durch die Ultraschallsonde (8, 10) während des Betriebs des lokalen Benutzers zu aktivieren und zu deaktivieren.

2. Ultraschallbildgebungssystem nach Anspruch 1, wobei die Ultraschallabtastvorrichtung ferner so angepasst ist, dass sie auf Befehle reagiert, die von dem entfernten Ort gesendet werden, um die Ultraschallabtastvorrichtung zu steuern.

3. Ultraschallbildgebungssystem nach Anspruch 2, wobei die Aktivierungs- und Deaktivierungsbefehlssignale digitale Befehle sind, die von dem entfernten Ort gesendet werden, um die Übertragung von Ultraschallenergie durch die Ultraschallabtastvorrichtung zu aktivieren und zu deaktivieren.

4. Ultraschallbildgebungssystem nach Anspruch 3, wobei die Ultraschallabtastvorrichtung ferner einen Befehlsdecoder (34) umfasst, der auf digitale Befehle reagiert, die von dem entfernten Ort gesendet und angepasst werden, um die Identität empfangener Befehle zu bestimmen.

5. Ultraschallbildgebungssystem nach Anspruch 2, wobei die Ultraschallabtastvorrichtung ferner so angepasst ist, dass sie auf verbale Befehle reagiert, die von dem entfernten Ort gesendet werden, um es der Ultraschallabtastvorrichtung zu ermöglichen, Ultraschallenergie für das sofortige Abtastverfahren zu übertragen.

6. Ultraschallbildgebungssystem nach Anspruch 1, wobei die Ultraschallabtastvorrichtung ferner eine Sendesteuerung (18) umfasst, die mit der Ultraschallsonde (8, 10) gekoppelt und angepasst ist, um auf den Empfang der von dem entfernten Ort initiierten Aktivierungs- und Deaktivierungssignale zu reagieren, um die Übertragung von Ultraschallenergie durch die Ultraschallsonde zu aktivieren und zu deaktivieren.

7. Ultraschallbildgebungssystem nach Anspruch 6, wobei die Ultraschallsonde (8, 10) ferner eine Wandleranordnung (12) umfasst, die zum Übertragen von Ultraschallenergie unter Steuerung des Sendecontrollers (18) angepasst ist.

8. Ultraschallbildgebungssystem nach Anspruch 1, wobei die Ultraschallabtastvorrichtung ferner ein MODEM/WiFi-Radio (130) umfasst, das angepasst ist, um Bilder an den entfernten Ort zu senden und Steuerbefehle von dem entfernten Ort zu empfangen.

9. Ultraschallbildgebungssystem nach Anspruch 1, wobei die Ultraschallabtastvorrichtung ferner eine zellulare Netzwerkschaltung (110) umfasst, die angepasst ist, um Bilder an den entfernten Ort zu senden und Steuerbefehle von dem entfernten Ort zu empfangen.

10. Ultraschallbildgebungssystem nach Anspruch 1, wobei die Ultraschallabtastvorrichtung ferner für die Videokommunikation mit dem entfernten Ort angepasst ist.

11. Ultraschallbildgebungssystem nach Anspruch 10, wobei die Ultraschallabtastvorrichtung ferner eine Smartphone-Kamera (118) oder eine Webcam umfasst.

12. Ultraschallbildgebungssystem nach Anspruch 1, wobei die Ultraschallabtastvorrichtung ferner für die Audiokommunikation mit dem entfernten Ort angepasst ist.

13. Ultraschallbildgebungssystem nach Anspruch 12, wobei die Ultraschallabtastvorrichtung ferner einen Lautsprecher (114) und ein Mikrofon (116) umfasst, wobei die Ultraschallabtastvorrichtung vorzugweise ferner ein Ultraschallbildaufnahmeprogramm umfasst, Ultraschallbildaufnahmeprogramm, das angepasst ist, um vom entfernten Ort Aktiviert oder Deaktiviert werden.

14. Ultraschallbildgebungssystem nach Anspruch 1, wobei die Ultraschallsonde (8, 10) ferner eine Wandleranordnung (12) und eine Hochspannungsversorgung umfasst, die selektiv mit der Wandleranordnung gekoppelt ist,
wobei die Hochspannungsversorgung angepasst ist, um die Wandleranordnung unter der Steuerung von dem entfernten Ort selektiv zu koppeln.

15. Computerimplementiertes Verfahren zur Fernsteuerung eines Abtastverfahrens mittels eines Ultraschallbildgebungssystems, umfassend:
Koppeln einer Ultraschallabtastvorrichtung mit einer lokalen Anzeige- und Benutzersteuervorrichtung (100) zur Steuerung durch einen lokalen Benutzer;
kontrollierbare Übertragung von Ultraschallenergie mittels einer Ultraschallsonde (8, 10), die zur kontrollierbaren Übertragung von Ultraschallenergie geeignet ist;
mittels eines Ultraschallbildprozessors (32) und einer Kommunikationseinheit, Senden von Bilddaten zu und Empfangen von Steuersignalen von einem entfernten Ort, der mit der Ultraschallabtastvorrichtung über eine Netzwerkverbindung (40) verbunden ist;
Anzeigen der von der Ultraschallabtastvorrichtung empfangenen Bilddaten mittels einer Ultraschallbildanzeige (108), die sich an dem entfernten Ort befindet;
Reagieren durch die Ultraschallabtastvorrichtung auf Steuersignale, die von dem entfernten Ort initiiert wurden, einschließlich einer Aktivierung und Deaktivierung des Befehlssignals, das konfiguriert ist, um die Übertragung von Ultraschallenergie durch die Ultraschallsonde (8, 10) während des Betriebs des lokalen Benutzers zu aktivieren und zu deaktivieren.

## Revendications

1. Système d'imagerie par ultrasons pour la commande à distance d'une procédure de balayage comprenant:
un dispositif de balayage à ultrasons adapté pour être accouplable avec un dispositif d'affichage local et de commande d'utilisateur (100) pour la commande par un utilisateur local, et comprenant une sonde à ultrasons (8, 10) adaptée pour transmettre de manière commandable une énergie ultrasonore, un processeur d'image à ultrasons (32), et une unité de communication adaptée pour envoyer des données d'image vers et recevoir des signaux de commande depuis un emplacement distant connecté au dispositif de balayage à ultrasons via une connexion de réseau (40);
un afficheur d'image à ultrasons (108) qui est situé à l'emplacement distant et adapté pour afficher les données d'image reçues du dispositif de balayage à ultrasons, où le dispositif de balayage à ultrasons est adapté pour répondre aux signaux de commande lancés à partir de l'emplacement distant, y compris un signal de commande d'activation et de désactivation configuré pour activer et désactiver la transmission d'énergie ultrasonore par la sonde ultrasonore (8, 10) pendant l'opération de l'utilisateur local.

2. Système d'imagerie par ultrasons selon la revendication 1, dans lequel le dispositif de balayage à ultrasons est en outre adapté pour répondre à des commandes envoyées de l'emplacement distant pour commander le dispositif de balayage à ultrasons.

3. Système d'imagerie par ultrasons selon la revendication 2, dans lequel les signaux de commande d'activation et de désactivation sont des commandes numériques envoyées de l'emplacement distant pour activer et désactiver la transmission d'énergie ultrasonore par le dispositif de balayage à ultrasons.

4. Système d'imagerie par ultrasons selon la revendication 3, dans lequel le dispositif de balayage à ultrasons comprend en outre un décodeur de commande (34) qui répond aux commandes numériques envoyées de l'emplacement distant et adapté pour déterminer l'identité des commandes reçues.

5. Système d'imagerie par ultrasons selon la revendication 2, dans lequel le dispositif de balayage à ultrasons est en outre adapté pour répondre à des commandes verbales envoyées de l'emplacement distant pour permettre au dispositif de balayage à ultrasons de transmettre l'énergie ultrasonore pour la procédure de balayage instantané.

6. Système d'imagerie par ultrasons selon la revendication 1, dans lequel le dispositif de balayage à ultrasons comprend en outre un dispositif de commande de transmission (18) qui est couplé à la sonde à ultrasons (8, 10) et adapté pour répondre à la réception des signaux d'activation et de désactivation lancés de l'emplacement distant pour activer et désactiver la transmission d'énergie ultrasonore par la sonde à ultrasons.

7. Système d'imagerie par ultrasons selon la revendication 6, dans lequel la sonde ultrasonore (8, 10) comprend en outre un réseau de transducteurs (12) adapté pour transmettre de l'énergie ultrasonore sous la commande du dispositif de commande de transmission (18) .

8. Système d'imagerie par ultrasons selon la revendication 1, dans lequel le dispositif de balayage à ultrasons comprend en outre une radio MODEM/WiFi (130) adaptée pour envoyer des images vers l'emplacement distant et recevoir des instructions de commande depuis l'emplacement distant.

9. Système d'imagerie par ultrasons selon la revendication 1, dans lequel le dispositif de balayage à ultrasons comprend en outre une circuiterie de réseau cellulaire (110) adaptée pour envoyer des images vers l'emplacement distant et recevoir des instructions de commande depuis l'emplacement distant.

10. Système d'imagerie par ultrasons selon la revendication 1, dans lequel le dispositif de balayage à ultrasons est en outre adapté pour une communication vidéo avec l'emplacement distant.

11. Système d'imagerie à ultrasons selon la revendication 10, dans lequel le dispositif de balayage à ultrasons comprend en outre une caméra de smartphone (118) ou une webcam.

12. Système d'imagerie par ultrasons selon la revendication 1, dans lequel le dispositif de balayage à ultrasons est en outre adapté pour une communication audio avec l'emplacement distant.

13. Système d'imagerie par ultrasons selon la revendication 12, dans lequel le dispositif de balayage à ultrasons comprend en outre un haut-parleur (114) et un microphone (116), où de préférence le dispositif de balayage à ultrasons comprend en outre un programme d'acquisition d'images à ultrasons qui est adapté pour être activé ou désactivé de l'emplacement distant.

14. Système d'imagerie par ultrasons selon la revendication 1, dans lequel la sonde à ultrasons (8, 10) comprend en outre un transducteur de réseau (12) et une alimentation à haute tension couplée sélectivement au transducteur de réseau,
où l'alimentation à haute tension est adaptée pour être sélectivement couplée au transducteur de réseau sous la commande de l'emplacement distant.

15. Procédé mis en œuvre par ordinateur pour la commande à distance d'une procédure de balayage au moyen d'un système d'imagerie à ultrasons, consistant à :
coupler un dispositif de balayage à ultrasons avec un dispositif d'affichage local et de commande d'utilisateur (100) pour la commande par un utilisateur local;
transmettre de manière commandable de l'énergie ultrasonore au moyen d'une sonde à ultrasons (8, 10) adaptée pour transmettre de manière commandable de l'énergie ultrasonore;
envoyer, au moyen d'un processeur d'image à ultrasons (32) et d'une unité de communication, des données d'image vers et recevoir des signaux de commande depuis un emplacement distant connecté au dispositif de balayage à ultrasons via une connexion de réseau (40);
afficher les données d'image reçues du dispositif de balayage à ultrasons au moyen d'un afficheur d'image à ultrasons (108) qui est situé à l'emplacement distant;
répondre, par le dispositif de balayage à ultrasons, pour commander des signaux lancés de l'emplacement distant y compris un signal de commande d'activation et de désactivation configuré pour activer et désactiver la transmission d'énergie ultrasonore par la sonde ultrasonore (8, 10) pendant l'opération de l'utilisateur local.
